# EUROPEAN PATENT APPLICATION

(11) **EP 4 623 823 A1**
(43) Date of publication of application: **01.10.2025**
(21) Application number: 24767207.4
(22) Date of filing: 07.03.2024
(51) Int. Cl.: A61B 5/151, A61B 5/107, A61M 5/42, A61M 5/158

(54) **PIERCING SYSTEM, INFORMATION PROCESSING METHOD, COMPUTER PROGRAM, AND INFORMATION PROCESSING DEVICE**

(30) Priority: 07.03.2023 JP 2023034567
(71) Applicant: Terumo Kabushiki Kaisha, Tokyo 151-0072 (JP)
(72) Inventor: TSUMAKI, Shota, Nakakoma-gun, Yamanashi 409-3853 (JP)
(74) Representative: KIPA AB
(86) International application number: PCT/JP2024/008708
(87) International publication number: WO 2024/185841

(57) **Abstract**

Provided is a puncture system and the like capable of easily switching a display mode of a blood vessel visualized image.

A puncture system includes: a blood vessel visualization device capable of displaying a blood vessel visualized image in which a blood vessel of a living body is visualized; and a puncture tool including a marker, in which the blood vessel visualization device acquires a marker state in the puncture tool, and specifies a display mode of a blood vessel visualized image based on the acquired marker state.

## Description

### Technical Field

The present invention relates to a puncture system, an information processing method, a computer program, and an information processing apparatus.

### Background Art

In a medical site, puncture work on a subject is performed for blood sampling, injection, continuous infusion, indwelling of an intravascular catheter, and the like. Medical personnel punctures a body surface of a subject up to a target position in a blood vessel, with a puncture tool such as a needle or a catheter. In order to support such puncture work, a technique for visualizing a blood vessel of a subject has been proposed.

For example, Patent Literature 1 discloses a blood vessel position display device capable of displaying an image indicating a position of a blood vessel suitable for puncture with a needle.

### Citation List

### Patent Literature

Patent Literature 1: JP 2020-185198 A

### Summary of Invention

### Technical Problem

There is a need to switch a display mode of a blood vessel visualized image in displaying the blood vessel visualized image in which a blood vessel is visualized. For example, in a case where a blood vessel visualized image in which a blood vessel is visualized is displayed on a surface of an arm of a subject, the blood vessel visualized image is displayed before puncture, and the blood vessel visualized image is not displayed when puncture is performed so that it is easy to observe skin deformation and the like. Although it is conceivable that an operator switches between display and non-display of the blood vessel visualized image by operating a switch of a blood vessel visualization device that displays the blood vessel visualized image, it is not easy to perform such a switch operation in a state of gripping a puncture tool.

An object of the present disclosure is to provide a puncture system and the like capable of easily switching a display mode of a blood vessel visualized image.

### Solution to Problem

(1) A puncture system according to one aspect of the present disclosure includes: a blood vessel visualization device capable of displaying a blood vessel visualized image in which a blood vessel of a living body is visualized; and a puncture tool including a marker, in which the blood vessel visualization device acquires a marker state in the puncture tool, and specifies a display mode of a blood vessel visualized image based on the acquired marker state.
(2) In the puncture system according to (1) above, the marker state may include a size of a range of the marker.
(3) In the puncture system according to (1) or (2) above, an image obtained by imaging the marker may be acquired, and the display mode may be specified based on a size of a range of the marker in the acquired image.
(4) The puncture system according to any one of (1) to (3) above, the display mode may include display or non-display of the blood vessel visualized image.
(5) The puncture system according to any one of (1) to (4) above, the display mode may include at least one of color, brightness, or contrast of the blood vessel visualized image.
(6) The puncture system according to any one of (1) to (5) above, the marker state may be classified into three or more, and the display mode that is different may be set for each marker state.
(7) The puncture system according to any one of (1) to (6) above, the marker may be provided near a portion to be gripped by a user in the puncture tool.
(8) The puncture system according to any one of (1) to (7) above, the marker may emit, fluoresce, or reflect near-infrared light.
(9) The puncture system according to any one of (1) to (8) above, the blood vessel visualization device may include: a light source that emits light of a specific wavelength; an imaging device that images a blood vessel of the living body and the marker; and a display device that displays the blood vessel visualized image.
(10) In an information processing method according to one aspect of the present disclosure, a computer executes processing including: acquiring a marker state in a puncture tool that includes a marker; and specifying a display mode of a blood vessel visualized image in which a blood vessel of a living body is visualized, based on the acquired marker state.
(11) A computer program according to one aspect of the present disclosure causes a computer to execute processing including: acquiring a marker state in a puncture tool that includes a marker; and specifying a display mode of a blood vessel visualized image in which a blood vessel of a living body is visualized, based on the acquired marker state.
(12) An information processing apparatus according to one aspect of the present disclosure includes a control unit that executes processing including: acquiring a marker state in a puncture tool that includes a marker; and specifying a display mode of a blood vessel visualized image in which a blood vessel of a living body is visualized, based on the acquired marker state.

### Advantageous Effects of Invention

According to the present disclosure, a display mode of a blood vessel visualized image can be easily switched.

### Brief Description of Drawings

Fig. 1 is a schematic view of a puncture system according to a first embodiment.
Fig. 2 is an explanatory view for explaining an exemplary configuration of an indwelling needle of the first embodiment.
Fig. 3 is a block diagram illustrating an exemplary configuration of an information processing apparatus.
Fig. 4 is a flowchart illustrating an example of a processing procedure executed by the information processing apparatus.
Fig. 5 is an explanatory view for explaining an exemplary configuration of an indwelling needle of a second embodiment.
Fig. 6 is a block diagram illustrating an exemplary configuration of an information processing apparatus according to the second embodiment.
Fig. 7 is a table illustrating a content example of information stored in a display mode DB.
Fig. 8 is a flowchart illustrating an example of a processing procedure executed by the information processing apparatus according to the second embodiment.

### Description of Embodiments

The present disclosure is specifically described below with reference to the drawings illustrating embodiments.

### (First Embodiment)

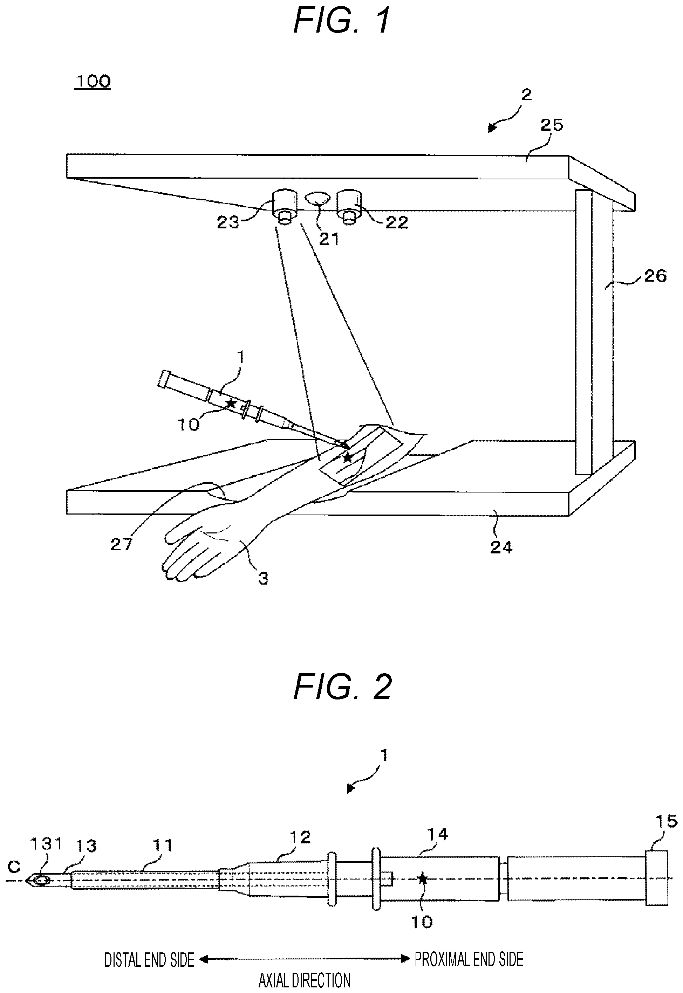

Fig. 1 is a schematic view of a puncture system 100 according to a first embodiment. The puncture system 100 includes an indwelling needle 1 and an information processing apparatus 2. The indwelling needle 1 is an example of a puncture tool. Hereinafter, as illustrated in Fig. 1, a case where a puncture target site is a part of a forearm of a living body (subject) 3 will be described as an example.

The indwelling needle 1 is, for example, a peripheral arteriovenous indwelling needle. An operator performs vascular puncture on the living body 3 with the indwelling needle 1. The operator is an example of a user of this system. The indwelling needle 1 is provided with a marker 10. The marker 10 is a label that functions as a switch for instructing a display mode of a blood vessel visualized image to be described later, and includes, for example, a phosphor.

The information processing apparatus 2 is provided in a facility such as a medical institution or an inspection institution where puncture work is performed. The information processing apparatus 2 functions as a marker recognition device that detects a state of the marker 10 provided to the indwelling needle 1, and as a blood vessel visualization device that detects a blood vessel of the living body 3 and visualizes the detected blood vessel. The information processing apparatus 2 displays a blood vessel visualized image in which a blood vessel is visualized, in accordance with a display mode corresponding to a detection result of the state of the marker 10.

The information processing apparatus 2 includes a light source 21, a camera 22, a display device 23, and the like. In the example illustrated in Fig. 1, the information processing apparatus 2 includes a bottom surface portion 24, a top surface portion 25, and a support column 26 connecting the bottom surface portion 24 and the top surface portion 25. Both the bottom surface portion 24 and the top surface portion 25 have a rectangular shape extending in a horizontal direction, and are disposed so as to be perpendicular to the support column 26. The bottom surface portion 24 and the top surface portion 25 overlap each other as viewed in a vertical direction. For example, the information processing apparatus 2 is installed and used on a horizontal table such as a treatment table at the time of use. Although Fig. 1 illustrates the fixed information processing apparatus 2, the information processing apparatus 2 may be portable or may be configured to be grippable by the operator.

On an upper surface of the bottom surface portion 24, a recessed portion 27 is formed. The recessed portion 27 has a shape on which the arm of the subject, which is the living body 3, can be placed, and a posture and a position of the arm are fixed by the subject placing the arm on the recessed portion 27 at the time of puncture. The bottom surface portion 24 may include a fixing member such as a fixing belt, and may be configured to be able to more reliably fix the position of the living body 3. Note that the bottom surface portion 24 does not need to include the recessed portion 27.

The light source 21, the camera 22, and the display device 23 are provided above the recessed portion 27, on a lower surface of the top surface portion 25. The light source 21, the camera 22, and the display device 23 are provided side by side in a longitudinal direction, for example. The camera 22 is provided immediately above the puncture target site, that is, at a position facing the puncture target site. The camera 22 is fixed to the top surface portion 25 so as to include, in an imaging region, a periphery of the blood vessel as the puncture target site and the indwelling needle 1 that is to puncture the blood vessel. The camera 22 is preferably fixed to the top surface portion 25 in a state where a position and an angle can be adjusted, so that the camera 22 is disposed immediately above the puncture target site in accordance with a running state of the blood vessel and movement of the indwelling needle 1.

The light source 21 emits light of a specific wavelength on the living body 3 and the marker 10. The light source 21 may emit, for example, near-infrared light. In consideration of absorption by hemoglobin in blood, an emission wavelength of the light source 21 can be set to, for example, 600 nm to 2500 nm, preferably 700 nm to 1400 nm, and more preferably 780 nm to 940 nm. The light source 21 may include a first light source 21 that emits light having a first wavelength on the marker 10 and a second light source 21 that emits light having a second wavelength different from the first wavelength on the living body 3. Note that, natural light, a fluorescent lamp, or the like (not illustrated) may be used as the light source 21 to emit visible light on at least one of the marker 10 or the living body 3.

The camera 22 is an imaging device including a lens, an imaging element, and the like. The camera 22 receives transmitted (or reflected) light of light emitted from the light source 21, acquires a near-infrared imaging signal, and generates a captured image as near-infrared image data on the basis of the acquired near-infrared imaging signal. The camera 22 may acquire a visible imaging signal, and generate a captured image as visible image data on the basis of the acquired visible imaging signal. The captured image obtained by the camera 22 is an image obtained by imaging the puncture target site and the indwelling needle 1 from above. In a case where the blood vessel of the puncture target site and the marker 10 are present in the imaging region of the camera 22, the captured image includes a blood vessel visualized image representing the blood vessel of the puncture target site and a marker image representing the marker 10.

A spectral sensitivity wavelength range of the camera 22 is not particularly limited as long as the spectral sensitivity wavelength range corresponds to an emission wavelength of the light source 21, and can be appropriately set in consideration of reaction wavelengths of the marker 10 and the blood vessel. As an example, it is assumed that a reaction wavelength related to the marker 10, that is, a wavelength of light emitted by the marker 10 is 400 to 600 nm. Since the reaction wavelength related to the blood vessel of the living body 3, that is, a wavelength of light absorbed by the blood vessel is about 800 nm to 900 nm, the spectral sensitivity wavelength range of the camera 22 can be set to 400 to 2500 nm including both of these reaction wavelengths, and is preferably 400 to 1000 nm. In this case, a captured image is obtained in which the marker 10, the blood vessel, and other portions are represented with different levels of luminance. The camera 22 may have spectral sensitivity with respect to RGB, and may be capable of detecting each color signal of RGB.

The camera 22 may include a camera 22 for detecting the marker 10 having a spectral sensitivity wavelength corresponding to the reaction wavelength of the marker 10, and a camera 22 for detecting a blood vessel having a spectral sensitivity wavelength corresponding to the reaction wavelength of the blood vessel. In this case, a marker captured image including a marker image is generated by the camera 22 for detecting the marker 10, and a blood vessel captured image including a blood vessel visualized image is generated by the camera 22 for detecting a blood vessel. The spectral sensitivity wavelength ranges in the camera 22 for detecting the marker 10 and the camera 22 for detecting the blood vessel may be the same or partially overlap, or may be different from each other.

Note that installation positions of the light source 21 and the camera 22 are only required to be positions where the blood vessel and the marker 10 can be detected, and are not limited to those in which the light source 21 and the camera 22 are installed on the same side with respect to a detection target. For example, the light source 21 and the camera 22 may be installed on sides facing each other with the detection target interposed therebetween, and the light source 21 and the camera 22 on the same side with respect to the detection target and the light source 21 and the camera 22 facing each other with the detection target interposed therebetween may be used in combination.

The display device 23 is an output device for presenting, to the operator, a blood vessel visualized image in which a blood vessel of the living body 3 is visualized. The display device 23 of the present embodiment includes a projector, and projects an image on a body surface of the living body 3. The display device 23 may include, for example, a display apparatus such as goggles, glasses, or a head-mounted display that can be worn by the operator. The display device 23 is preferably fixed to the top surface portion 25 in a state where a position and an angle can be adjusted, so that the display device 23 is disposed above the puncture target site in accordance with a running state of the blood vessel and movement of the indwelling needle 1.

The configuration of the information processing apparatus 2 is not limited to the example illustrated in Fig. 1, and may be an appropriate configuration as long as the information processing apparatus 2 can function as the marker recognition device and the blood vessel visualization device. Furthermore, in the present embodiment, an example has been described in which the information processing apparatus 2 functions as the marker recognition device and the blood vessel visualization device, but the marker recognition device and the blood vessel visualization device are not limited to one common information processing apparatus 2, and may be provided as separate devices.

Fig. 2 is an explanatory view for explaining an exemplary configuration of the indwelling needle 1 of the first embodiment. Hereinafter, various configurations and the like will be described with a direction along an axial center C of the indwelling needle 1 as an axial center direction, a left side in Fig. 2 as a "distal end side", and a right side as a "proximal end side".

The indwelling needle 1 includes the marker 10, a catheter 11, a catheter hub 12 coupled to a proximal end of the catheter 11, an inner needle (needle) 13 inserted through inside the catheter 11, an inner needle hub 14 coupled to a proximal end of the inner needle 13, and a filter 15 coupled to a proximal end of the inner needle hub 14. To the proximal end of the inner needle hub 14, a syringe (not illustrated) can be connected. In the catheter hub 12, a hemostatic valve (not illustrated) may be included. The indwelling needle 1 does not need to include the filter 15.

The catheter 11 constitutes an outer needle and reaches the vicinity of a distal end of the inner needle 13. From a distal end of the catheter 11, a blade surface 131 formed at the distal end of the inner needle 13 is exposed (protrudes). When the distal end of the inner needle 13 is inserted into the blood vessel, the catheter 11 is also inserted into the same blood vessel.

The marker 10 is provided in the vicinity of a portion to be gripped by the operator in the indwelling needle 1. The vicinity of the portion to be gripped by the operator means, for example, a range that a finger of one hand reaches when the operator grips the indwelling needle 1 with the one hand for puncture. That is, the marker 10 is disposed at a position where an operation on the marker 10 can be executed with one hand in a state where the indwelling needle 1 is gripped by the one hand. The marker 10 is preferably provided on the proximal end side of the indwelling needle 1. In the example illustrated in Fig. 2, the portion to be gripped by the operator is the inner needle hub 14, and the marker 10 is provided on an outer peripheral surface of the inner needle hub 14. The marker 10 is formed at a position that is, when the blade surface 131 is directed upward, directed similarly upward. According to the above configuration, when the indwelling needle 1 that is used with the blade surface 131 facing upward at the time of puncture is imaged from above, a captured image in which the marker 10 is more clearly recognized can be obtained.

A shape of the marker 10 is not particularly limited, and may be an appropriate shape such as a circle, a triangle, a square, a star, and a cross. Since the marker 10 has a complicated shape, a possibility of erroneous detection can be reduced, which is preferable. Fig. 2 illustrates the marker 10 having a star shape. The marker 10 may be formed over a predetermined length in a circumferential direction of the indwelling needle 1 or may be formed over the entire circumference. When the marker 10 is formed in the circumferential direction, the shape of the marker 10 may be, for example, a linear shape such as one line, two lines, or a wavy line. The shape of the marker 10 may be set according to a type of the indwelling needle 1.

The marker 10 includes, for example, a near-infrared fluorescent dye that emits near-infrared fluorescence of a specific wavelength by irradiation with excitation light. The camera 22 receives the fluorescence of the marker 10 to detect a shape, a range (region), and the like of the marker 10.

The marker 10 may include, for example, a near-infrared fluorescent dye that emits near-infrared fluorescence having a wavelength of 600 nm to 2500 nm, and may include a fluorescent dye that emits fluorescence in a wavelength range (for example, a wavelength of 400 to 600 nm) corresponding to green, blue, or the like that does not exist in the living body of the subject. By making the reaction wavelength of the marker 10 different from the reaction wavelength of the blood vessel of the living body 3, the marker 10 and the blood vessel can be clearly distinguished and detected.

The marker 10 may be formed, for example, by including a fluorescent dye in a base material by kneading, coating, or the like, and the formed marker 10 may be attached to a predetermined location of the indwelling needle 1**.** The marker 10 may be formed by directly including a fluorescent dye in a predetermined location of the indwelling needle 1 by kneading, coating, or the like. The marker 10 may be attached to the indwelling needle 1 at a manufacturing stage, or may be attached by the operator or the like before use of the indwelling needle 1**.** The marker 10 may be made of a removable material such as a seal member.

Note that the configuration and the detection method of the marker 10 are not limited as long as the marker 10 can be detected by the camera 22. The marker 10 may include, for example, a light emitter that emits light having a specific wavelength, an upconversion (UC) phosphor that emits light having a specific wavelength, a reflector that reflects emitted light having a specific wavelength, and the like. The marker 10 may have a reflection structure that reflects light, by performing surface treatment such as embossing processing or unevenness processing. The marker 10 includes, for example, a material having visible light absorbency, such as a colored ink, and may be detected by a visible light camera.

The indwelling needle 1 is not limited to the above example, and may have an appropriate configuration as long as the indwelling needle 1 is a puncture tool to be inserted into a blood vessel of the living body 3. The indwelling needle 1 may be a dialysis indwelling needle, a peripherally inserted central catheter (PICC), a midline catheter, a central venous catheter (CVC), or the like. Like a blood sampling device, the puncture tool may not include a catheter and may include a puncture needle (inner needle), a syringe, and the like. According to the medical device described above, the marker 10 is provided in the vicinity of the portion to be gripped by the operator.

A puncturing method using the indwelling needle 1 will be described. The operator grips the indwelling needle 1 including the inner needle 13, punctures a surface of the living body 3 (a skin surface of the subject) toward a blood vessel, and gradually inserts the inner needle 13 toward a desired site. As a result, the distal end of the inner needle 13 advances while cutting and opening a body tissue. After the inner needle 13 punctures the target blood vessel, the catheter 11 and the catheter hub 12 are moved to the distal end to insert the catheter 11 into the blood vessel. After the insertion, by removing and the inner needle 13, the inner needle hub 14, and the filter 15 while leaving the catheter 11 and the catheter hub 12, the catheter 11 and the catheter hub 12 are indwelled in the blood vessel.

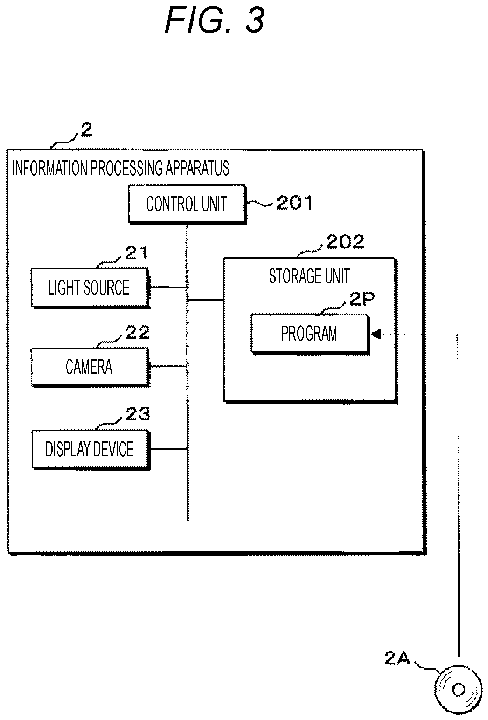

Fig. 3 is a block diagram illustrating an exemplary configuration of the information processing apparatus 2. The information processing apparatus 2 is a computer, and includes a control unit 201 and a storage unit 202 in addition to the light source 21, the camera 22, and the display device 23 described above. Each unit is connected by a bus. The information processing apparatus 2 may be a multi-computer including a plurality of computers or may be a virtual machine virtually constructed by software.

The control unit 201 includes one or a plurality of arithmetic processing devices such as a central processing unit (CPU), a micro-processing unit (MPU), and a graphics processing unit (GPU). The control unit 201 controls each component by using a memory such as a read only memory (ROM) or a random access memory (RAM), a clock, a counter, and the like that are incorporated, to execute processing.

The storage unit 202 includes, for example, a nonvolatile memory such as a hard disk, a flash memory, or a solid state drive (SSD). The storage unit 202 may be an external storage device connected to the information processing apparatus 2. The storage unit 202 stores various computer programs and data referred to by the control unit 201. The storage unit 202 stores a program 2P for causing a computer to execute processing related to output of a blood vessel visualized image. The storage unit 202 may further store a reaction wavelength related to the marker 10, a reaction wavelength related to a blood vessel, a threshold to be used for various determinations described later, and the like, as data necessary for executing the program 2P.

The computer program (computer program product) including the program 2P may be provided using a non-transitory recording medium 2A on which the computer program is recorded in a readable manner. The storage unit 202 stores a computer program read from the recording medium 2A by using a reading device (not illustrated). The recording medium 2A is, for example, a magnetic disk, an optical disk, a semiconductor memory, or the like. In addition, the computer program may be downloaded from an external server connected to a communication network, and stored in the storage unit 202. The program 2P may include a single computer program or a plurality of computer programs, and may be executed on a single computer or may be executed on a plurality of computers interconnected by a communication network.

The configuration of the information processing apparatus 2 is not limited to the above-described example, and may include, for example, an operation unit for receiving an operation performed by a user, a communication unit for communicating with an external device via a network such as the Internet, an input/output unit for connecting an external device, and the like. Furthermore, a part of the information processing apparatus 2 such as the control unit 201 and the storage unit 202 may be configured as a server computer or the like in a place away from the facility such as the medical institution where puncture work is performed. In this case, the control unit 201 may receive a captured image obtained by the camera 22 through communication, and transmit information corresponding to the received captured image to the display device 23 through communication to display the information.

The information processing apparatus 2 generates a display image including a blood vessel visualized image and a marker image, on the basis of a captured image captured by the camera 22 at the time of puncture. Specifically, by extracting a blood vessel and the marker 10 in the captured image, a display image is generated including a blood vessel visualized image indicating a region corresponding to the blood vessel and a marker image indicating a region corresponding to the marker 10. A method for extracting the blood vessel and the marker 10 in the captured image is not limited, but a detection target and a position (range) thereof may be specified on the basis of a luminance value of the image, and a method such as pattern matching may be used. The information processing apparatus 2 may identify the blood vessel and the marker 10 in the image data on the basis of known reaction wavelengths of the blood vessel and the marker 10.

When the captured marker image and the captured blood vessel image are individually generated by the camera 22, a display image including a blood vessel visualized image and a display image including a marker image may be individually generated as display images, or one display image obtained by combining the blood vessel visualized image and the marker image obtained from individual captured images may be generated. The display image may be any image as long as at least a blood vessel visualized image is included, and may be configured not to include a marker image. Note that the display image is not limited to an image obtained by performing predetermined image processing on a captured image, and may be the captured image itself. In a case where the blood vessel or the marker 10 is not present in an imaging region of the camera 22, the captured image and the display image are also images not including the blood vessel visualized image or the marker image.

The information processing apparatus 2 causes the display device 23 to display the generated display image. At this time, the information processing apparatus 2 determines whether or not the display image needs to be displayed in accordance with a state of the marker 10 operated by the operator, and switches between display and non-display of the display image in accordance with the determination result.

The information processing apparatus 2 of the present embodiment determines whether or not the display image needs to be displayed on the basis of the presence or absence of the marker 10 in the captured image. In a case where the marker 10 is included in the captured image, that is, in a case where a size of a range of the marker 10 in the captured image is not zero, it is determined that the display image needs to be displayed. In a case where the marker 10 is not included in the captured image, that is, in a case where the size of the range of the marker 10 in the captured image is zero, it is determined that there is no need to display the display image. In a case where the size of the range of the marker 10 in the captured image is equal to or larger than a preset threshold, it may be determined that the marker 10 is included in the captured image and the display image needs to be displayed. In a case where it is determined that the display image needs to be displayed, the information processing apparatus 2 causes the display image to be displayed via the display device 23. In a case where it is determined that there is no need to display the display image, the information processing apparatus 2 does not cause the display image to be displayed.

The operator instructs the information processing apparatus 2 to switch the display mode by performing a predetermined operation on the marker 10 and changing a state of the marker 10 detected by the camera 22. Specifically, an operation of changing the size of the range of the marker 10 is performed. The operator starts puncture work while gripping the indwelling needle 1 that includes the marker 10. At a stage before puncturing the surface of the living body 3 with the inner needle 13, blood vessel running can be grasped more accurately by projecting the blood vessel visualized image onto the surface of the living body 3. In this way, in a case where the operator desires the blood vessel visualized image to be displayed, the operator does not perform an operation on the marker 10. When no operation is performed on the marker 10, substantially the entire shape of the marker 10 is shown in the captured image in a recognizable manner. Therefore, it is determined that the display image needs to be displayed, and the display image is displayed.

Whereas, at a stage where a puncture position is determined on the basis of blood vessel running and the surface of the living body 3 is punctured with the inner needle 13, the surface of the living body 3 can be easily viewed by not projecting the blood vessel visualized image. As described above, in a case where the operator does not desire the blood vessel visualized image to be displayed, the operator performs a predetermined operation on the marker 10. For example, the operator covers the marker 10 with a finger from above such that the marker 10 is not imaged by the camera 22, to cause the marker 10 of the indwelling needle 1 to disappear. As a result, the range of the marker 10 captured by the camera 22 changes. When the operation on the marker 10 is performed, a captured image not including the marker 10 is acquired. Therefore, it is determined that there is no need to display the display image, and the display image is not displayed. When the display image is desired to be displayed again, the state of the display image can be switched to the display state by moving the finger covering the marker 10 from above the marker 10 to end the operation on the marker 10.

The method for switching between display and non-display of the display image using the marker 10 is not limited to the above example. For example, the information processing apparatus 2 may switch the state of the display image from the current state to another state in response to detection of an operation on the marker 10. The information processing apparatus 2 receives a display mode switching instruction by acquiring a captured image that does not include the marker 10 in accordance with the operation performed by the operator on the marker 10. The information processing apparatus 2 determines whether or not the display image need to be displayed on the basis of a current state of the display image. In a case where the current state of the display image is the display state, it is determined that there is no need to display the display image, and the state is switched to the non-display state.

After the display mode of the display image is switched and the operation on the marker 10 is released, the operator again performs the operation on the marker 10. The information processing apparatus 2 receives a display mode switching instruction by acquiring a captured image that does not include the marker 10 in accordance with the operation on the marker 10. In a case where the current state of the display image is the non-display state, it is determined the display image needs to be displayed, and the display state is switched to the display state. In this manner, the display mode of the display image may be specified according to the time-series state of the marker 10.

The information processing apparatus 2 may specify a duration indicating a predetermined state of the marker 10 together with the state of the marker 10, on the basis of the captured images acquired in time series. The duration may be obtained by specifying time during which captured images not including the marker 10 are continuously acquired. For example, in a case where a length of the duration is equal to or longer than a threshold, the information processing apparatus 2 may determine that the display mode switching instruction has been received.

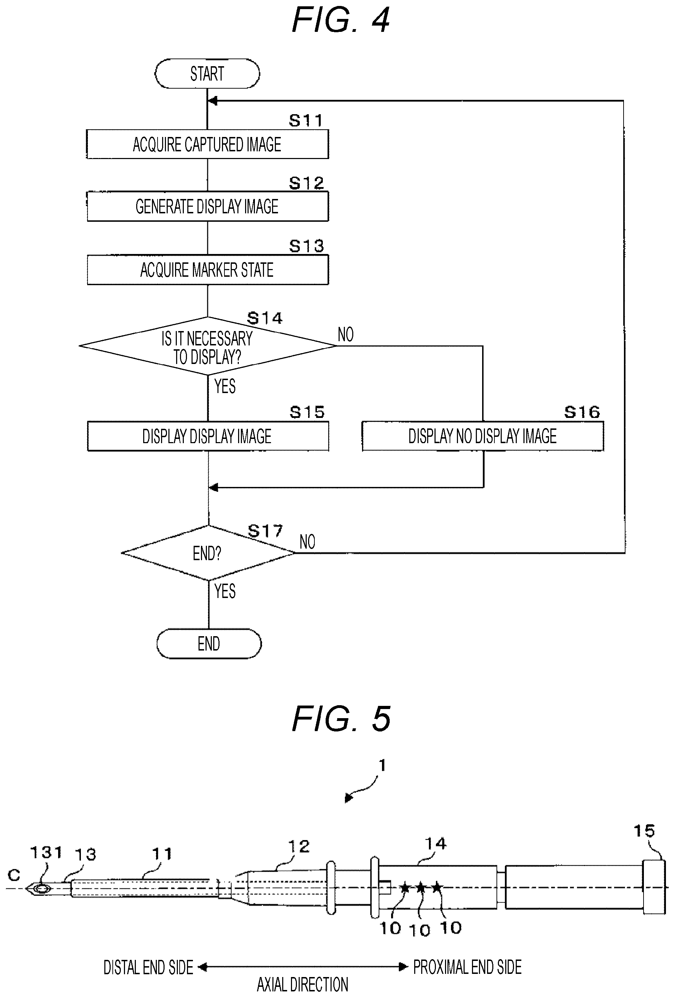

Fig. 4 is a flowchart illustrating an example of a processing procedure executed by the information processing apparatus 2. Processing in each flowchart below may be executed by the control unit 201 in accordance with the program 2P stored in the storage unit 202 of the information processing apparatus 2, may be achieved by a dedicated hardware circuit (for example, FPGA or ASIC) provided in the control unit 201, or may be achieved by a combination thereof.

The control unit 201 of the information processing apparatus 2 acquires a captured image captured by the camera 22 (step S11). The control unit 201 executes image analysis on the acquired captured image to recognize a blood vessel and the marker 10 in the captured image, and generates a display image including a blood vessel visualized image and a marker image (step S12). The display image generation processing may be performed after it is determined that display is necessary by display necessity determination processing to be described later.

The control unit 201 acquires a state of the marker 10 on the basis of the acquired captured image (step S13). Specifically, the control unit 201 acquires the presence or absence of the marker 10 in the captured image as the state of the marker 10. The control unit 201 may specify a duration of the state of the marker 10 on the basis of the captured images acquired in time series.

The control unit 201 determines whether or not the display image need to be displayed on the basis of the acquired marker state 10 (step S14). The determination processing in step S14 corresponds to specifying processing of the display mode of the display image. When it is determined that the display image needs to be displayed because the captured image includes the marker 10 (step S14: YES), the control unit 201 outputs the display image to the display device 23 to display the display image (step S15).

When it is determined that there is no need to display the display image because the marker 10 is not included in the captured image (step S14: NO), the control unit 201 does not output the display image to the display device 23 and does not cause the display image to be displayed (step S16).

The control unit 201 determines whether or not to end the processing (step S17). For example, when it is determined not to end the processing because a predetermined end operation has not been executed (step S17: NO), the control unit 201 returns the processing to step S11. The control unit 201 acquires a new captured image, and repeatedly executes switching of the display mode of the display image on the basis of the state of the marker 10 in the acquired captured image. When it is determined to end the processing because the predetermined end operation has been executed (step S17: YES), the control unit 201 ends the series of processing.

According to the present embodiment, display and non-display of the display image can be switched according to the state of the marker 10 in the captured image. By switching the display mode of the display image, it is possible to improve visibility of the puncture target site for the operator, and it is possible to immediately present the blood vessel visualized image when necessary, and to perform puncture more accurately. By providing the marker 10 in the vicinity of the portion of the indwelling needle 1 to be gripped by the operator, the operator can easily perform an operation on the marker 10 without using the other hand while gripping the indwelling needle 1 with one hand. Since the operation on the marker 10 can be executed with one hand, the display state can be suitably switched without hindering a procedure even in the middle of the procedure using both hands.

By forming the marker 10 with a light emitter that emits near-infrared light, the marker 10 can be detected together with the blood vessel by using the light source 21 and the camera 22 which are used to generate the blood vessel visualized image, so that the puncture system 100 can be further simplified. By configuring the marker 10 to be detachable, the marker 10 can be repeatedly used, and economic efficiency is enhanced.

By presenting the marker image together with the blood vessel visualized image by using the display image, it is possible to visually and clearly grasp the state of the marker 10 functioning as a reception unit that receives a switching instruction for the display mode, and thus, it is possible to reliably perform the operation on the marker 10.

The puncture system 100 more preferably uses a projector, goggles worn by the operator, or the like as the display device 23, but the display device 23 is not limited thereto, and may be a display apparatus provided at a predetermined location without being worn by the operator.

### (Second Embodiment)

In a second embodiment, a configuration for specifying a larger number of display modes on the basis of a marker state 10 will be described. In the embodiment below, differences from the first embodiment are mainly described. The same components as those of the first embodiment are denoted by the same reference numerals as those used in the first embodiment, and detailed explanation thereof will not be repeated.

Fig. 5 is an explanatory view for explaining an exemplary configuration of an indwelling needle 1 of the second embodiment. As illustrated in Fig. 5, the indwelling needle 1 of the second embodiment includes a plurality of markers 10. Note that, in the present embodiment, the number of markers 10 is three. The plurality of markers 10 are arranged in a line along an axial direction of the indwelling needle 1. Note that, the number of markers 10 may be two or four or more. In addition, an arrangement position of the marker 10 is not particularly limited as long as the arrangement position is in the vicinity of a portion to be gripped by an operator in the indwelling needle 1 and all the markers 10 can be operated by the operator with one hand.

The markers 10 each may be configured to be distinguishable from each other, for example, by making a difference in shape, size, direction, line type, and the like for each marker 10. In addition, reaction wavelengths of the markers 10 may be the same or different. By making the reaction wavelengths of the markers 10 different from each other, it is easy to identify each marker 10.

In the present embodiment, more display modes are set in accordance with states of the plurality of markers 10 indicated by the plurality of markers 10. The states of the marker 10 can be set by, for example, the number of markers 10 recognized with a captured image. In the present embodiment, four types of display modes are set individually in association with zero to three markers 10.

The display mode includes normal display in which a normal display image is displayed, brightness change display in which an image whose brightness has been changed is displayed, color reversal display in which an image whose color has been reversed is displayed, and non-display in which no display image is displayed. In the brightness change display, brightness of a blood vessel visualized image in a normal display image may be changed to be larger or smaller by a predetermined value. In the color reversal, pixel values corresponding to a blood vessel visualized image and a marker image in a normal display image and pixel values corresponding to other regions may be reversed. In addition, the display mode may include a saturation change display for displaying an image in which saturation in a normal display image is changed by a predetermined value, a contrast change display for displaying an image in which contrast in a normal display image is changed by a predetermined value, and the like.

The display modes such as the color reversal, the brightness change, and the contrast change may be further classified into a plurality of stages according to a degree of reversal and a degree of change. For example, regarding the brightness change, three types of display modes of weak brightness, medium brightness, and strong brightness may be further set.

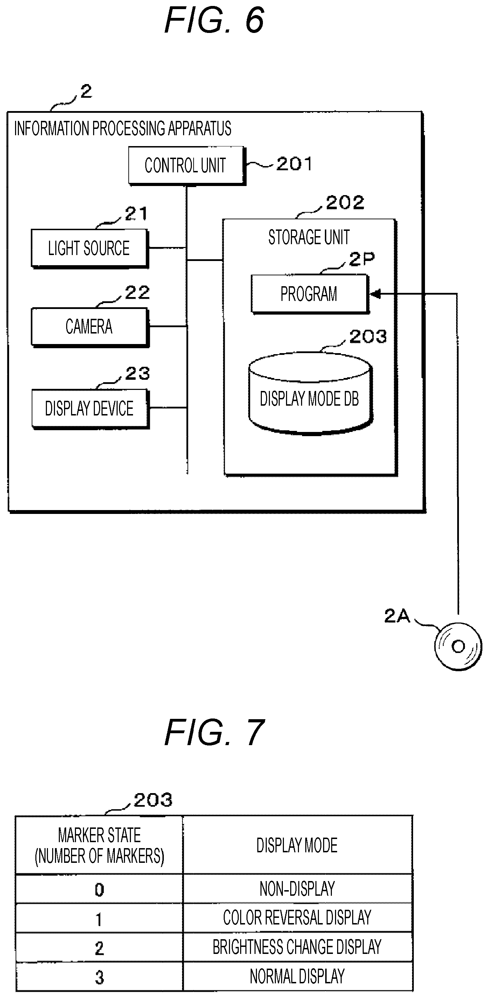

Fig. 6 is a block diagram illustrating an exemplary configuration of an information processing apparatus 2 of the second embodiment. A storage unit 202 of the information processing apparatus 2 in the second embodiment stores a display mode database (DB) 203 as data necessary for executing a program 2P. The display mode DB 203 is a database that stores information regarding a display mode.

Fig. 7 is a table illustrating a content example of information stored in the display mode DB 203. The display mode DB 203 stores, for example, a record in which information such as a state and a display mode of the marker 10 is associated. In the example illustrated in Fig. 7, the state of the marker 10 is represented by the number of recognized markers 10, and includes the numbers of 0 to 3. The display mode includes the above-described four types of display modes (normal display, brightness change display, color reversal display, and non-display). As illustrated in Fig. 7, different display modes are set individually for the numbers of markers 10.

When the operator desires to change the display mode to a predetermined display mode, for example, the number of markers 10 recognized by a camera 22 is changed by covering a specific number of markers 10 with a finger, among the plurality of markers 10 provided on the indwelling needle 1. By specifying a position, a range, and the like of the marker 10 included in a captured image on the basis of the captured image obtained by imaging the indwelling needle 1, the information processing apparatus 2 acquires the number of recognizable markers 10. The information processing apparatus 2 may acquire the number of markers 10 by specifying the number of ranges in which a size of a range recognized as the marker 10 is equal to or larger than a predetermined value in the captured image. The information processing apparatus 2 specifies the display mode in accordance with the number of markers 10 in the captured image on the basis of the information stored in the display mode DB 203. The information processing apparatus 2 changes the display state of the display image in accordance with the specified display mode.

The state of the marker 10 is not limited to the state that is set in accordance with the number of markers 10. The state of the marker 10 may be classified by, for example, a plurality of thresholds set for a size of a range of the marker 10. In this case, the state of the marker 10 may be specified by obtaining a total value of ranges of the markers 10 from the captured image, and comparing the obtained total value with the threshold for the state of each marker 10.

The state of the marker 10 may be specified by a change amount from a start of puncture, with reference to the state of the marker 10 at the start of puncture. The display mode DB 203 may store a threshold related to an amount of change in a size of a range of the marker 10.

The marker 10 is not limited to the plurality of markers 10, and may be any marker as long as operation states in a plurality of stages can be detected. For example, one marker 10 may be formed over a relatively wide range, and the state of the marker 10 may be classified on the basis of a change in size of the range of the marker 10 caused by an operation performed by the operator on the marker 10. The operator operates the marker 10 so as to obtain a desired display mode by adjusting a degree of covering the marker 10 with a finger.

The state of the marker 10 may be set further in consideration of a duration of the state of the marker 10. For example, the information processing apparatus 2 specifies a state of the marker 10 and a duration of the state in unit time on the basis of captured images acquired in time series, thereby obtaining the number of times of detection of the state of the marker 10 per unit time. The display mode may be changed according to the obtained number of times of detection and the state of the marker 10.

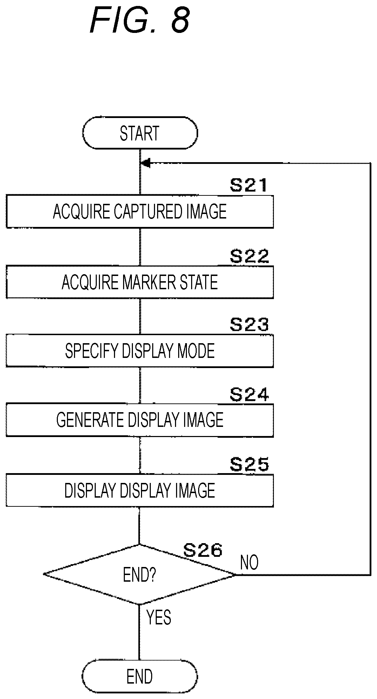

Fig. 8 is a flowchart illustrating an example of a processing procedure executed by the information processing apparatus 2 according to the second embodiment.

A control unit 201 of the information processing apparatus 2 acquires a captured image captured by the camera 22 (step S21). The control unit 201 acquires a state of the marker 10 on the basis of the acquired captured image (step S22). Specifically, the control unit 201 acquires the number of markers 10 included in the captured image, by performing image analysis on the acquired captured image and recognizing the markers 10 in the captured image. The control unit 201 may specify a duration of the state of the marker 10 on the basis of the captured images acquired in time series.

The control unit 201 specifies a display mode according to the acquired number of markers 10 on the basis of the information stored in the display mode DB 203 (step S23).

The control unit 201 generates a display image according to the specified display mode (step S24). The control unit 201 executes image analysis on the acquired captured image to recognize a blood vessel and the marker 10 in the captured image, and generates a normal display image including a blood vessel visualized image and a marker image. In addition, the control unit 201 performs image processing such as color reversal, brightness change, and contrast change on the generated normal display image as necessary on the basis of the specified display mode.

The control unit 201 outputs the generated display image to the display device 23 to display the display image (step S25). Note that, in a case where non-display is specified as the display mode, the processing of steps S24 to S25 may be omitted.

The control unit 201 determines whether or not to end the processing (step S26). For example, when it is determined not to end the processing (step S26: NO), the control unit 201 returns the processing to step S21. When it is determined to end the processing (step S26: YES), the control unit 201 ends the series of processing.

According to the present embodiment, since the switching operation of the display mode with the marker 10 can be applied to more various display modes, convenience of the system is improved. By setting a plurality of types of states of the marker 10 and setting various display modes for the individual states of the markers 10, the display modes can be easily switched according to the states of the markers 10. By considering the duration of the state of the marker 10, it is possible to apply to switching of more display modes. The marker 10 having a simple configuration can function as a reception unit that receives instructions for a plurality of types of display modes.

It should be understood that the embodiments disclosed herein are illustrative in all respects and are not restrictive. The technical features described in the examples can be combined with each other, and the scope of the present invention is intended to include all modifications within the scope of the claims and the scope equivalent to the claims.

The sequences in each of the embodiments are not limited, and each processing procedure may be carried out in a changed order, or a plurality of processes may be performed in parallel, within a range without contradictions. The processing subject in each process is not limited, and processes to be performed by the respective devices may be performed by some other device within a range without contradictions.

The matters described in each embodiment can be combined with each other. In addition, some or all of the independent claims and their dependent claims described in the claims can be combined together, regardless of their dependent relationships. Furthermore, although a form (multiple dependent claim form) in which a claim dependent on two or more other claims is described is used in the claims, the claim form is not limited thereto. The present invention may be described by using a form in which a multiple dependent claim dependent on at least one multiple dependent claim is described.

### Reference Signs List

- 100: Puncture system
- 1: Indwelling needle
- 10: Marker
- 11: Catheter
- 12: Catheter hub
- 13: Inner needle
- 14: Inner needle hub
- 15: Filter
- 2: Information processing apparatus
- 21: Light source
- 22: Camera (imaging device)
- 23: Display device
- 201: Control unit
- 202: Storage unit
- 2P: Program
- 2A: Recording medium
- 203: Display mode DB
- 3: Living body

## Claims

1. A puncture system comprising:
a blood vessel visualization device capable of displaying a blood vessel visualized image in which a blood vessel of a living body is visualized; and a puncture tool including a marker, wherein
the blood vessel visualization device
acquires a marker state in the puncture tool, and
specifies a display mode of a blood vessel visualized image based on the acquired marker state.

2. The puncture system according to claim 1, wherein
the marker state includes a size of a range of the marker.

3. The puncture system according to claim 1 or 2, wherein
an image obtained by imaging the marker is acquired, and
the display mode is specified based on a size of a range of the marker in the acquired image.

4. The puncture system according to claim 1 or 2, wherein
the display mode includes display or non-display of the blood vessel visualized image.

5. The puncture system according to claim 1 or 2, wherein
the display mode includes at least one of color, brightness, or contrast of the blood vessel visualized image.

6. The puncture system according to claim 1 or 2, wherein
the marker state is classified into three or more, and the display mode that is different is set for each marker state.

7. The puncture system according to claim 1 or 2, wherein
the marker is provided near a portion to be gripped by a user in the puncture tool.

8. The puncture system according to claim 1 or 2, wherein
the marker emits, fluoresces, or reflects near-infrared light.

9. The puncture system according to claim 1 or 2, wherein
the blood vessel visualization device includes: a light source that emits light of a specific wavelength; an imaging device that images a blood vessel of the living body and the marker; and a display device that displays the blood vessel visualized image.

10. An information processing method in which a computer executes processing comprising:
acquiring a marker state in a puncture tool that includes a marker; and
specifying a display mode of a blood vessel visualized image in which a blood vessel of a living body is visualized, based on the acquired marker state.

11. A computer program causing a computer to execute processing comprising:
acquiring a marker state in a puncture tool that includes a marker; and
specifying a display mode of a blood vessel visualized image in which a blood vessel of a living body is visualized, based on the acquired marker state.

12. An information processing apparatus comprising
a control unit that executes processing comprising:
acquiring a marker state in a puncture tool that includes a marker; and
specifying a display mode of a blood vessel visualized image in which a blood vessel of a living body is visualized, based on the acquired marker state.
